# EUROPEAN PATENT SPECIFICATION

(11) **EP 1 034 761 B1**
(45) Date of publication and mention of the grant of the patent: **05.10.2005**
(21) Application number: 00302019.5
(22) Date of filing: 13.03.2000
(51) Int. Cl.: A61F 13/494

(54) **Disposable diaper with elastic barrier cuffs**
Wegwerfwindel mit elastischen dichten Rändern
Couche jetable comportant des barrières d'étanchéité élastiques

(30) Priority: 12.03.1999 JP 6675999
(43) Date of publication of application: 13.09.2000
(73) Proprietor: UNI-CHARM CORPORATION, Ehime-ken (JP)
(72) Inventor: Shimoe, Nariaki, c/o Technical Center, Mitoyo-gun, Kagawa-ken 769-1602 (JP); Jitoe, Yoshikazu, c/o Technical Center, Mitoyo-gun, Kagawa-ken 769-1602 (JP); Kumasaka, Yoshinori, c/o Technical Center, Mitoyo-gun, Kagawa-ken 769-1602 (JP)
(74) Representative: Parry, Christopher Stephen

(56) References cited:
- EP-A- 0 623 331
- EP-A- 0 692 233
- WO-A-01/05347
- WO-A-96/05792
- GB-A- 2 250 921
- US-A- 4 734 246
- US-A- 4 743 246
- US-A- 5 069 678
- US-A- 5 188 627
- US-A- 5 743 994
- US-A- 5 766 411

## Description

This invention relates to a disposable diaper for absorption and containment of excretion.

Japanese Patent Application Disclosure No. 1996-24291 describes a disposable diaper provided with elastic members associated with a pair of leg-openings, the elastic members comprising a first elastic member extending generally in U-shape along front halves of the leg-openings, a second elastic member extending generally in U-shape along rear halves of the leg-openings and a pair of third elastic members longitudinally extending to intersect the first and second elastic members in a longitudinally middle zone of a crotch region of the diaper. According to this Japanese Patent Application Disclosure No. 1996-24291, the first, second and third elastic members cooperate together to encircle the respective leg-openings and thereby to prevent excretion from leaking sideways. ,

Japanese Patent Application Disclosure No. 1996-322876 describes a disposable garment comprising a basic structure which comprises, in turn, a liquid-pervious topsheet, a liquid-impervious backsheet and a liquid-absorbent core disposed between these two sheets, and a pair of barrier cuffs longitudinally extending outside transversely opposite side edges of the absorbent core and normally biased to rise on the upper surface of the basic structure. Each of the barrier cuffs has a free side edge provided with an elastic member secured under tension thereto, a proximal side edge joined to the upper surface of the topsheet and longitudinally opposite ends collapsed inwardly of the garment and, in this state, joined to the upper surface of the topsheet. Contraction of the elastic member causes the barrier cuff to rise on the garment as the garment is curved with its inner surface inside. In order to alleviate irritate to the wearer's skin, the barrier cuffs are made of a hydrophobic nonwoven fabric or plastic film. According to this Japanese Patent Application Disclosure No. 1996-322876, the barrier cuffs function as preventing excretion from leaking sideways. Certainly a garment having the first, second and third elastic members cooperating together to encircle the respective leg-openings, on one hand, and the pair of barrier cuffs provided along their free side edges with the elastic members secured under tension thereto, on the other hand, can be supposed from a combination of the garments disclosed in the above-mentioned two applications.

However, the third elastic member is secured to the basic structure of the garment and cannot assist the barrier cuffs to rise on the garment. It is nothing but contraction of the elastic members secured to the respective free side edges of the barrier cuffs to cause the barrier cuffs to rise on the garment. If an effect biasing the barrier cuffs to rise on the garment is inadequate, the barrier cuffs will collapse and the level of their free side edges will be too low to prevent excretion from leaking sideways beyond the barrier cuffs.

It is an object of this invention to provide a disposable diaper provided with a pair of barrier cuffs adapted to be easily risen and to be reliably maintained in their risen positions so that excretion can be effectively prevented from leaking sideways.

According to this invention, there is provided a disposable diaper comprising: a laminated panel comprising a liquid-pervious topsheet, a liquid-impervious backsheet and a liquid-absorbent core disposed therebetween to form a front waist region, a rear waist region and a crotch region; the laminated panel being formed with a pair of gasket cuffs which laterally extend outward from transversely opposite side edges of the absorbent core, each of the gasket cuffs being provided at least longitudinally thereof with a first elastic member which comprises a first elastic sub-member extending on a front half of said laminated panel generally in U-shape with an intermediate portion thereof extending across said laminated panel in a longitudinally middle zone of said crotch region and a second elastic sub-member extending on a rear half of said laminated panel generally in U-shape with an intermediate portion thereof extending across said laminated panel in said middle zone but being spaced apart from said intermediate portion of said first elastic sub-member; characterised by the laminated panel further being provided adjacent the transversely opposite side edges of the absorbent core with a pair of barrier cuffs which extend longitudinally of the laminated panel and normally bias to rise on an upper surface of the laminated panel, each of the barrier cuffs having a free side edge and a proximal side edge joined to the upper surface of the laminated panel, the free side edge of the barrier cuff being provided longitudinally thereof with a second elastic member; and each of the barrier cuffs being provided between the free and proximal side edges with at least a single third elastic member longitudinally thereof at least across the crotch region; wherein each of said third elastic members is in two level crossing relationship with an intermediate portion of said first elastic member in said crotch region such that the third elastic members and first elastic member are spaced apart from each other in the vertical direction of the diaper when the barrier cuffs have risen up on the upper surface of the laminated panel and cross one another as viewed from above; and wherein the stretch stresses of said first and second elastic sub-members are greater than those of said second and third elastic members.

This invention comprises the following embodiments:

The third elastic members are secured to the barrier

cuffs in the vicinity of the proximal side edges of the barrier cuffs.

Each of the third elastic members extend in parallel to the second elastic members.

Longitudinally opposite ends of the barrier cuffs are joined to an upper surface of the laminated panel so as to be collapsed inwardly of the laminated panel.
Fig. 1 is a perspective view of a partially cutaway disposable diaper according to this invention;
Fig. 2 is a sectional view taken along a line A-A in Fig. 1; and
Fig. 3 is a sectional view taken along a line B-B in Fig.1.

Details of a disposable diaper according to this invention will be more fully understood from the description given hereunder with reference to the accompanying drawings.

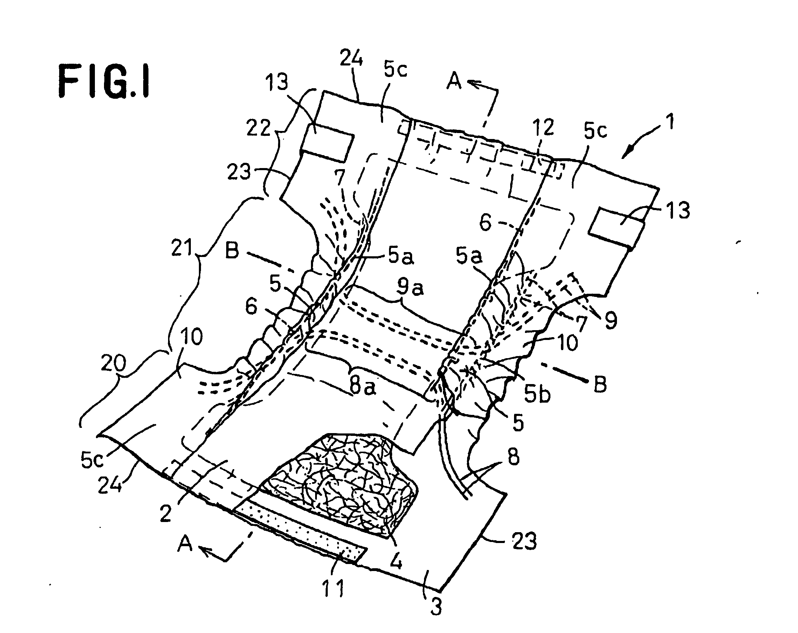

Fig. 1 is a perspective view of a partially cutaway disposable diaper comprising a laminated panel 1. The panel 1 comprises a liquid-pervious topsheet 2, a liquid-impervious backsheet 3 and a liquid-absorbent core 4 disposed between these two sheets 2, 3 and secured to the inner surface of at least one of these sheets 2, 3. The panel 1 has a front waist region 20, a rear waist region 22 and a crotch region 21 extending between these front and rear waist regions 20, 22 as viewed longitudinally of the panel 1, i.e. in the direction intersecting the transverse direction of the panel 1.

The panel 1 is formed with a pair of gasket cuffs 10 laterally extending outward from the transversely opposite side edges of the absorbent core 4 and each of these gasket cuffs 10 comprises a narrow zone extending in the crotch region 21 and relatively wide zones extending in the front and rear waist regions 20, 22. The gasket cuffs 10 have transversely opposite side edges 23 extending longitudinally of the panel 1 and curved inwardly of the panel 1 in the crotch region 21 and longitudinally opposite ends 24 extending transversely of the side edges 23.

The panel 1 is provided on its inner surface with a pair of barrier cuffs 5 extending immediately outside transversely opposite side edges of the absorbent core 4 longitudinally of the panel 1. Each of the barrier cuffs 5 is made of a nonwoven fabric sheet and has a free side edge 5a extending across the crotch region 21, a proximal side edge 5b extending longitudinally of the barrier cuff 5 and longitudinally opposite ends 5c lying in the front and rear waist regions 20, 22. A portion of the barrier cuff 5 extending transversely outward from the proximal side edge 5b inclusive of the longitudinally opposite ends 5c has the same shape as an outer side edge zone of the backsheet 3.

The barrier cuff 5 has the proximal side edge 5b joined to the upper surface of the topsheet 2 and its longitudinally opposite ends 5c collapsed inwardly of the panel 1 and, in this state, joined to the upper surface of the topsheet 2. The free side edge 5a of the barrier cuff 5 is provided with the elastic member 6 extending longitudinally of the barrier cuff 5 and secured under tension thereto.

The barrier cuff 5 is provided between the free side edge 5a and the proximal side edge 5b rather to the proximal side edge 5b with a pair of elastic members 7 extending longitudinally of the barrier cuff 5 across the crotch region 21 and secured under tension to the barrier cuff 5. These elastic members 7 extend neither into the front waist region 20 nor into the rear waist region 22. Fig. 1 shows the panel 1 as being curved longitudinally thereof with its inner surface inside and, in this state of the panel 1, contraction of the elastic members 6, 7 cause the barrier cuff 5 to rise on the panel 1. In this manner, the elastic member 6 cooperates with the pair of elastic members 7 to ensure that the barrier cuff 5 can be reliably risen on the upper surface of the panel 1.

It should be understood here that the pair of elastic members 7 may lie substantially in the middle between the free side edge 5a and the proximal side edge 5b of the barrier cuff 5 or slightly to said proximal side edge 5b. It is also possible, in an alternative embodiment, to provide the pair of elastic members 7 extending across not only the crotch region 21 but also into the front and rear waist regions 20, 22.

The panel 1 is provided with a generally U-shaped elastic sub-member 8 lying on a front half of the crotch region 21 and a generally U-shaped elastic sub-member 9 lying on a rear half of the crotch region 21. These elastic sub-members 8, 9 are intended to be associated with the leg-openings and secured under tension to the panel 1. The elastic sub-member 8 extends along the front half of the left leg-opening (i.e. the right leg-opening as viewed in Fig. 1), then across the longitudinally middle zone of the crotch region 21 and along the front half of the right leg-opening (i.e. the left leg-opening as viewed in Fig. 1). Similarly, the elastic member 9 extends along the rear half of the left leg-opening (i.e. the right leg-opening as viewed in Fig. 1), then across the longitudinally middle zone of the crotch region 21 and along the rear half of the right leg-opening (i.e. the left leg-opening as viewed in Fig. 1). In the longitudinally middle zone, these elastic sub-members 8, 9 generally describe U- and inverted U-shapes, respectively, and intermediate portions 8a, 9a of the respective elastic sub-members 8, 9 extending across the longitudinally middle zone are spaced apart from each other by a predetermined dimension. Transversely opposite ends of the intermediate portions 8a, 9a are in two level crossing relationship with the transversely opposite ends of the elastic members 7, respectively.

Each of the gasket cuffs 10 is formed by joining the portion of the barrier cuff 5 laterally extending outward from the proximal side edge 5b thereof and the associated side edge portion of the backsheet 3 to each other.

The front and rear waist regions 20, 22 of the panel 1 are provided along longitudinally opposite ends 24 of the panel 1 with tape-like elastic members 11, 12 extending transversely of the panel 1 so as to be associated with a waist-opening. These tape-like elastic members 11, 12 are disposed between the topsheet 2 and the backsheet 3 and secured under tension to at least one of these sheets 2, 3. Fig. 1 shows a state in which the elastic members 8, 9, 11, 12 have been slackened and, in consequence, gathers are formed along the longitudinally opposite ends 24 of the panel 1 and the transversely opposite side edges 23 of the panel 1 in the crotch region 21.

In the rear waist region 22, a pair of tape fasteners 13 have their proximal ends attached to the side flaps so that these tape fasteners 13 extend inwardly of the panel 1. These tape fasteners 13 have their free ends coated with an adhesive agent. In the front waist region 20, the panel 1 is provided on its outer surface with target tapes so that the fasteners 13 may be anchored on the respective target tapes an adhesive agent applied on the inner surfaces of the respective tape fasteners 13 to form the waist-opening and the pair of leg-openings (not shown).

As has been described above, the elastic member 7 provided adjacent the proximal side edge 5b of the barrier cuff 5 is in two level crossing relationship with the longitudinally opposite ends of the respective intermediate portions 8a, 9a of the elastic sub-members 8, 9 extending along the front half and the rear half of the associated one of the leg-openings. With this unique arrangement, these elastic members 7, 8, 9 cooperate together to encircle the wearer's legs when the diaper is put on the wearer's body. In this manner, an improved preventive effect is achieved against sideway leakage of excretion from the crotch region 21.

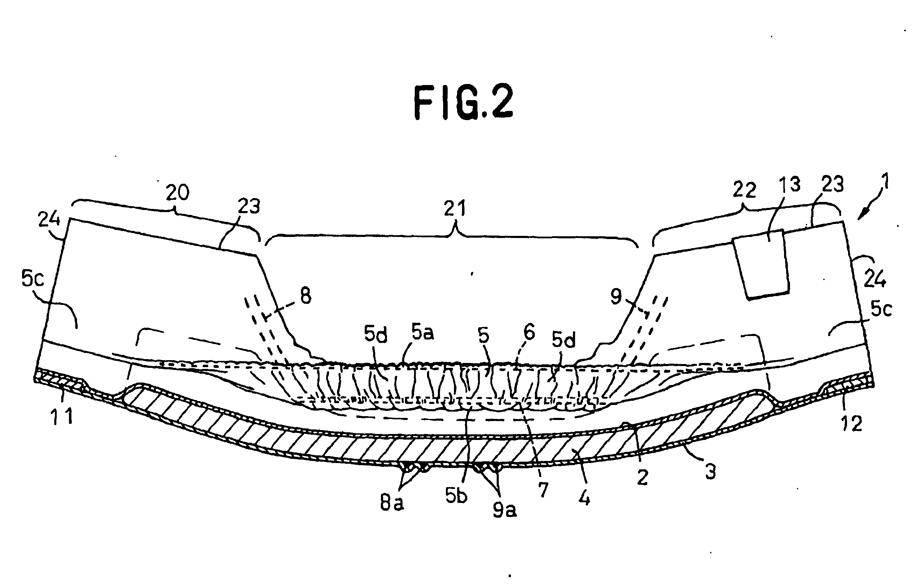

Fig. 2 is a sectional view taken along a line A - A in Fig. 1, showing the pair of barrier cuffs 5 rise on the upper surface of the panel 1. In this state, the pair of barrier cuffs 5 have been drawn toward the crotch region 21 under contraction of the elastic members 6, 7 and a plurality of gathers 5d vertically extending have been formed between the free side edges 5a and the proximal side edges 5b. These gathers 5d prevent the barrier cuffs 5 from being bent in the longitudinal direction between their free side edges 5a and proximal side edges 5b and, at the same time, prevent the barrier cuffs 5 from being readily collapsed. The portions of the elastic sub-members 8, 9 extending between the transversely opposite side edges of the absorbent core 4 are disposed between the backsheet 3 and the absorbent core 4 and secured to the backsheet 3.

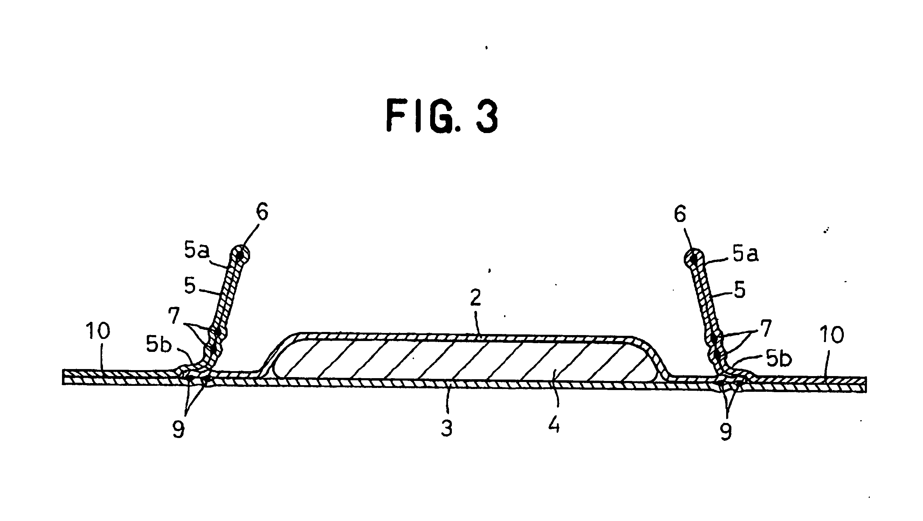

Fig. 3 is a sectional view taken along a line B - B in Fig. 1. On a point at which the elastic member 7 crosses the elastic sub-member 9, the elastic member 7 is spaced apart above from the elastic sub-member 9 by a predetermined dimension. Similarly, on a point at which the elastic member 7 crosses the elastic sub-member 8, the elastic member 7 is spaced apart above from the elastic sub-member 8 by a predetermined dimension (not shown).

In the panel 1, the transversely opposite side edges of the topsheet 2 terminate immediately outside the transversely opposite side edges of the absorbent core 4 and the transversely opposite side edges of the backsheet 3 extend outward beyond the side edges of the topsheet 2. The barrier cuffs 5 are folded inwardly of the panel 1 to wrap the elastic members 6, 7, respectively, which are secured to the barrier cuffs 5. Portions of the elastic sub-members 8, 9 extending outward beyond the transversely opposite side edges of the absorbent core 4 are disposed between the topsheet 2 and the backsheet 3 and secured to at least one of these sheets 2, 3.

The elastic members 6, 7 and the elastic members 8, 9 are of stretch stresses which are different one from another. The stretch stresses of these elastic members are arranged to be in the relationship that the stretch stresses of the elastic sub-members 8, 9 are greater than those of the elastic members 6, 7.

Stock material for the topsheet 2 may be selected from a group consisting of liquid-pervious sheets such as a nonwoven fabric and porous plastic film and, preferably a liquid-pervious hydrophobic sheet. Stock material for the backsheet 3 may be selected from a group consisting of a liquid-impervious plastic film, a laminate sheet of such plastic film and hydrophobic nonwoven fabric, and preferably a breathable liquid-impervious nonwoven fabric sheet. Stock material for the barrier cuffs 5 may be selected from a group consisting of a breathable nonwoven fabric and preferably a breathable liquid-impervious nonwoven fabric sheet. The nonwoven fabric may be selected from a group consisting of a spun bond nonwoven fabric, a spun lace nonwoven fabric and a melt blown nonwoven fabric each having a basis weight of 5-150 g/m². The absorbent core 4 is a mixture of fluff pulp and superabsorptive polymer particle compressed to a desired thickness and entirely covered with a liquid-pervious sheet such as tissue paper. Securing or mounting of the members may be performed using a suitable adhesive agent or glue, for example, a hot melt adhesive agent or heat-sealing technique.

This invention is applicable also to an open-type disposable diaper and the other pull-on type disposable diaper. Furthermore, this invention is not limited to the diaper in which the elastic members associated with the leg-openings describe U- and inverted U-shapes in the middle zone of the crotch region 21 and applicable also to the diaper in which the elastic members associated with the leg-openings extend along the transversely opposite side edges.

According to the undergarment of this invention, the elastic members secured to the barrier cuffs between their free side edges and proximal side edges assist the barrier cuffs to be reliably risen, prevent the barrier cuffs to be collapsed and cooperate with the elastic members associated with the leg-openings to encircle the wearer's legs. In this manner, a preventive effect against sideway leakage of excretion is remarkably improved.

## Claims

1. A disposable diaper comprising:
a laminated panel (1) comprising a liquid-pervious topsheet (2), a liquid-impervious backsheet (3) and a liquid-absorbent core (4) disposed therebetween to form a front waist region (20), a rear waist region (22) and a crotch region (21);
the laminated panel being formed with a pair of gasket cuffs (10) which laterally extend outward from transversely opposite side edges of the absorbent core, each of the gasket cuffs being provided at least longitudinally thereof with a first elastic member (8, 9) which comprises a first elastic sub-member (8) extending on a front half of said laminated panel generally in U-shape with an intermediate portion (8a) thereof extending across said laminated panel in a longitudinally middle zone of said crotch region and a second elastic sub-member (9) extending on a rear half of said laminated panel generally in U-shape with an intermediate portion (9a) thereof extending across said laminated panel in said middle zone but being spaced apart from said intermediate portion (8a) of said first elastic sub-member (8); **characterised by**
the laminated panel further being provided adjacent the transversely opposite side edges of the absorbent core with a pair of barrier cuffs (5) which extend longitudinally of the laminated panel and normally bias to rise on an upper surface of the laminated panel, each of the barrier cuffs having a free side edge (5a) and a proximal side edge (5b) joined to the upper surface of the laminated panel, the free side edge of the barrier cuff being provided longitudinally thereof with a second elastic member (6); and
each of the barrier cuffs being provided between the free and proximal side edges with at least a single third elastic member (7) longitudinally thereof at least across the crotch region; wherein each of said third elastic members (7) is in two level crossing relationship with an intermediate portion of said first elastic member (8, 9) in said crotch region such that the third elastic members (7) and first elastic member (8, 9) are spaced apart above from each other when the barrier cuffs (5) have risen up on the upper surface of the laminated panel and cross one another as viewed from above;
and wherein the stretch stresses of said first and second elastic sub-members (8, 9) are greater than those of said second and third elastic members (6, 7).

2. The diaper according to Claim 1, said third elastic members (7) are secured to said barrier cuffs in the vicinity of the proximal side edges of said barrier cuffs.

3. The diaper according to Claim 1, wherein each of said third elastic members (7) extends in parallel to said second elastic members (6).

4. The disposable diaper according to Claim 1, wherein longitudinally opposite ends of said barrier cuffs (5) are joined to an upper surface of said laminated panel so as to be collapsed inwardly of said laminated panel.

## Patentansprüche

1. Wegwerfwindel, aufweisend
eine laminierte Tafel (1) mit einer flüssigkeitsdurchlässigen oberen Lage (2), einer flüssigkeitsundurchlässigen rückwärtigen Lage (3) und einem dazwischen angeordneten flüssigkeitsabsorbierenden Kern (4) zur Bildung eines vorderen Taillenbereichs (20), eines hinteren Taillenbereichs (22) und eines Schrittbereichs (21),
wobei die laminierte Tafel mit einem Paar Dichtungsmanschetten (10) versehen ist, die von einander in Querrichtung gegenüberliegenden Seitenkanten des absorbierenden Kerns quer nach außen verlaufen und jeweils zumindest in ihrer Längsrichtung mit einem ersten elastischen Element (8, 9) versehen sind, das ein erstes elastisches Teilelement (8), das auf der vorderen Hälfte der laminierten Tafel im wesentlichen U-förmig verläuft und dessen Zwischenabschnitt (8a) in einer Längsmittelzone des Schrittbereichs über die laminierte Tafel hinweg verläuft, und ein zweites elastisches Teilelement (9) enthält, das auf der hinteren Hälfte der laminierten Tafel im wesentlichen U-förmig verläuft und dessen Zwischenabschnitt (9a) in der Mittelzone über die laminierte Tafel hinweg verläuft, von dem Zwischenabschnitt (8a) des ersten elastischen Teilelements (8) jedoch einen Abstand aufweist, **dadurch gekennzeichnet, daß**
die laminierte Tafel bei den einander in Querrichtung gegenüberliegenden Seitenkanten des absorbierenden Kerns außerdem mit einem Paar Sperrmanschetten (5) versehen ist, die in Längsrichtung der laminierten Tafel verlaufen, auf deren oberer Fläche normalerweise zum Aufrichten vorgespannt sind und jeweils eine freie Seitenkante (5a) und eine mit der oberen Fläche der laminierten Tafel verbundene nahe Seitenkante (5b) aufweisen, von denen die freie Seitenkante der Sperrmanschette in ihrer Längsrichtung mit einem zweiten elastischen Element (6) versehen ist, und
die Sperrmanschetten jeweils in ihrer Längsrichtung zwischen der freien und der nahen Seitenkante mindestens über den Schrittbereich hinweg mit mindestens einem einzelnen dritten elastischen Element (7) versehen sind, wobei die dritten elastischen Elemente jeweils eine kreuzende Beziehung in zwei Ebenen mit einem Zwischenabschnitt des ersten elastischen Elements (8, 9) im Schrittbereich aufweisen, so daß die dritten elastischen Elemente (7) und das erste elastische Element (8, 9) nach oben einen Abstand voneinander aufweisen, wenn die Sperrmanschetten (5) auf der oberen Fläche der laminierten Tafel aufstehen, und einander bei Blick von oben kreuzen,
wobei die Dehnungsspannungen des ersten und des zweiten elastischen Teilelements (8, 9) größer als die des zweiten und des dritten elastischen Elements (6, 7) sind.

2. Windel nach Anspruch 1, wobei die dritten elastischen Elemente (7) in der Gegend der nahen Seitenkanten der Sperrmanschetten an den Sperrmanschetten befestigt sind.

3. Windel nach Anspruch 1, wobei die dritten elastischen Elemente (7) jeweils parallel zu den zweiten elastischen Elementen (6) verlaufen.

4. Windel nach Anspruch 1, wobei einander in Längsrichtung gegenüberliegende Enden der Sperrmanschetten (5) an einer oberen Fläche der laminierten Tafel so befestigt sind, daß sie zum inneren der laminierten Tafel hin zusammenfallen.

## Revendications

1. Couche jetable comprenant :
un panneau stratifié (1) comprenant une feuille supérieure (2) perméable aux liquides, une feuille postérieure (3) imperméable aux liquides et un coeur (4) absorbant les liquides disposé entre celles-ci de manière à constituer une région de taille antérieure (20), une région de taille postérieure (22) et une région d'entrejambes (21) ;
le panneau stratifié étant constitué avec une paire de revers d'étanchéité (10) qui s'étendent latéralement vers l'extérieur à partir des bords latéraux transversalement opposés du coeur absorbant, chacun des revers étanches étant muni au moins longitudinalement par rapport à ceux-ci d'un premier élément élastique (8, 9), qui comprend un premier sous-élément élastique (8) s'étendant sur une moitié antérieure dudit panneau stratifié en forme générale de U, avec une partie intermédiaire (8a) de celui-ci s'étendant au travers dudit panneau stratifié dans une zone longitudinalement médiane de ladite région d'entre-jambes, et un deuxième sous-élément élastique (9) s'étendant sur une moitié postérieure dudit panneau stratifié en forme générale de U, avec une partie intermédiaire (9a) de celui-ci s'étendant au travers dudit panneau stratifié dans ladite zone médiane, mais avec un espacement vis-à-vis de ladite partie intermédiaire (8a) dudit premier sous-élément élastique (8), **caractérisée en ce que**
le panneau stratifié est en outre muni, au voisinage des bords latéraux transversalement opposés du coeur absorbant, d'une paire de revers formant barrière (5), qui s'étendent longitudinalement par rapport au panneau stratifié et sont normalement sollicités de manière à s'élever sur une surface supérieure du panneau stratifié, chacun des revers formant barrière ayant un bord latéral (5a) qui est libre et un bord latéral proximal (5b) qui est assemblé avec la surface supérieure du panneau stratifié, le bord latéral libre du revers formant barrière étant muni longitudinalement par rapport à celui-ci d'un deuxième élément élastique (6) ; et
chacun des revers formant barrière étant muni, entre les bords latéraux libre et proximal, d'au moins un troisième unique élément élastique (7), longitudinalement par rapport à celui-ci, au moins au travers de la région d'entre-jambes ; dans lequel chacun desdits troisièmes éléments élastiques (7) est en relation de croisement à deux niveaux avec une partie intermédiaire dudit premier élément élastique (8, 9) dans ladite région d'entrejambes, de sorte que les troisièmes éléments élastiques (7) et le premier élément élastique (8, 9) sont espacés au-dessus l'un de l'autre quand les revers formant barrière (5) se sont élevés sur la surface supérieure du panneau stratifié, et se croisent l'un l'autre quand on les regarde du dessus ;
et dans laquelle les contraintes d'étirement desdits premier et deuxième sous-éléments élastiques (8, 9) sont supérieures à celles desdits deuxième et troisième éléments élastiques (6, 7).

2. Couche selon la revendication 1, dans laquelle lesdits troisièmes éléments élastiques (7) sont fixés auxdits revers formant barrière au voisinage des bords latéraux proximaux desdits revers formant barrière.

3. Couche selon la revendication 1, dans laquelle chacun desdits troisièmes éléments élastiques (7) s'étend parallèlement auxdits deuxièmes éléments élastiques (6).

4. Couche selon la revendication 1, dans laquelle des extrémités longitudinalement opposées desdits revers formant barrière (5) sont assemblées à une surface supérieure dudit panneau stratifié de manière à être écrasées vers l'intérieur dudit panneau stratifié.
